(19) Europäisches Patentamt
European Patent Office
Office européen des brevets

(11) **EP 2 335 760 A1**

(12) **EUROPEAN PATENT APPLICATION**

(43) Date of publication:
**22.06.2011 Bulletin 2011/25**

(51) Int Cl.:
***A61M 16/10*** *(2006.01)*

(21) Application number: **09179796.9**

(22) Date of filing: **18.12.2009**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(71) Applicant: **General Electric Company**
**Schenectady, NY 12345 (US)**

(72) Inventors:
• **Lähde, Niina**
**02760, Espoo (FI)**

• **Holopainen, Timo**
**00510, Helsinki (FI)**

(74) Representative: **Kanerva, Arto**
**GE Healthcare Finland Oy**
**P.O. Box 900**
**00031 GE Helsinki (FI)**

(54) **Assembly, method and breathing circuit arrangement for removing moisture of breathing gas**

(57) An assembly (30) for removing a moisture of a breathing gas by transferring a thermal energy of said breathing gas is disclosed herein. The assembly includes a first thermally conductive element (31) in a thermal contact with the breathing gas flowing along a tube (17) absorbing a thermal energy of the breathing gas, and a medium (33) inside a space (32) being in thermal contact with the first thermally conductive element and which medium is able to absorb and transfer the thermal energy absorbed by the first thermally conductive elements. The assembly also includes a second thermally conductive element (34) being in a thermal contact with the medium absorbing the thermal energy transferred by the medium and releasing the thermal energy outside the tube. A method and a breathing circuit arrangement for removing a moisture of a breathing gas is also provided.

FIG. 2

## Description

BACKGROUND OF THE INVENTION

This disclosure relates generally to an assembly and method for removing a moisture of a breathing gas by transferring a thermal energy of said breathing gas flowing along a tube. This disclosure also relates to a breathing circuit arrangement for removing a moisture of a breathing gas when guiding the breathing gas exhaled and inhaled by a patient.

A patient breathing circuit can be a re-breathing circuit or an open circuit. The re-breathing circuit with a CO2 absorber is used when volatile anesthetic or NO2, or both, exist in anesthesia. Instead the open circuit is used with an intravenous (IV) anesthesia.

Inhalation anesthesia is thus delivered using a re-breathing circuit comprising an inspiratory limb through which a patient gets an inspired breathing gas from a ventilator, an expiratory limb carrying an exhaled gas back to the ventilator, a Y-piece connecting the inspiratory and expiratory limbs to a further patient limb providing a gas communication pathway to patient lungs. An expired gas comprises a lot of expensive and environment-hostile anesthesia gases. The re-breathing circuit is used to return the expired gas to next inspiration. Before doing this, the gas must be cleared out from the carbon dioxide extracted from the patient lungs as waste product of a patient metabolism and filled with oxygen and the anesthetic agent corresponding to the gas consumption. Gases are added through fresh gas inlet, which is used to control a re-breathing circuit mixture. Another port of the circuit is connected to a ventilator controlling a circuit pressure to ventilate the patient. Through this port the ventilator provides an inspiration volume that is for a typical adult patient 500 mL into the breathing circuit during the inspiration and receives a respective volume during the expiration. The ventilator comprises a volume that preserves the expiration gas for the next inspiration. Typically this ventilator preserving volume is maximum 1500 mL.

The clearance is done in a CO2 absorber comprising typically NaOH or KOH soda-lime. While absorbent removes CO2 from the breathing gases, it same time creates moisture and heat. Reaction happens in three phases. One molecule of carbon dioxide gives 4 molecules water.

(i) $CO_2 + H_2O <-> H_2CO_3$

(ii) $2 H_2CO_3 + 2 NaOH -> Na_2CO_3 + 4 H_2O + Heat$

(iii) $2 Ca(OH)_2 + Na_2CO_3 -> 2 CaCO_3 + 2 NaOH + Heat$

Breathing gas, when it exits the absorber, is approx. 45°C, and 100% saturated. Breathing gas encounters the room temperature and starts to cool down, and on this account the moisture starts to condensate. In state of art devices, condensation happens slowly, and unintentionally resulting to water condensate in patient breathing hoses. This increases breathing resistance, and also impairs ventilation functionalities. The worst case is that, water accumulates inside the breathing circuit where it cannot be noticed, and then not removed. If tidal volume for ventilation is 500 ml and respiration rate is 12. And temperature difference is from 45 °C to 22 °C. Total calculated amount of condensed water vapor is approximately 20 ml per hour. If patient breathing hoses diameter is 22 mm, and shape is parabola, it takes little over an hour, when water closes the airway. This point resistance of gas flow is quite high, because gas has to work its way through water.

During long anesthesia, which may take 2-3 hours, clinician needs to drain water from the hoses. For that, breathing hoses needs to disconnect. This disconnection cause great leak in circuit. Breathing gas flows to the atmosphere, and patient is without ventilation that time. The hospital personnel exposes to breathing gas, which also includes anesthesia agent. So disconnection of breathing hose is harmful for both, patient and personnel and great risk for patient safety.

It is well known to cool the breathing gas by conducting the gas through a pipe system comprising many thin plastic tubes where the water vapor is condensed to water. However, a resistance in breathing circuit increases, because of these long and narrow tubes. This also increases compliance of the breathing circuit by adding more volume to the circuit. This system is expensive to do and also creates sealing problems.

Also it is possible to cool the breathing gas actively by means of electrical components requiring electricity which is not desired. Further the breathing gas can be heated to avoid condensing the water, but again this requires electrical components and energy having the same problems as actively cooling the gas.

BRIEF DESCRIPTION OF THE INVENTION

The above-mentioned shortcomings, disadvantages and problems are addressed herein which will be understood by reading and understanding the following specification.

In an embodiment, an assembly for removing a moisture of a breathing gas by transferring a thermal energy of the breathing gas includes a first thermally conductive element in a thermal contact with the breathing gas flowing along a tube absorbing a thermal energy of the breathing gas and a medium inside a space being in thermal contact with the first thermally conductive element and which medium is able to absorb and transfer the thermal energy absorbed by the first thermally conductive element. The assembly for removing a moisture of a breathing gas also includes a second thermally conductive element being in a thermal contact with the medium absorbing the thermal energy transferred by the medium and releasing the thermal energy outside the tube.

In another embodiment, a method for removing a moisture of a breathing gas by transferring a thermal energy of the breathing gas flowing along a tube includes absorbing a thermal energy of the breathing gas flowing along the tube into a first thermally conductive element and absorbing from the first thermally conductive element the thermal energy to a medium transferring the thermal energy towards a second thermally conductive element. The method for removing a moisture of a breathing gas also includes absorbing from the medium the thermal energy to a second thermally conductive element and releasing the thermal energy of the second thermally conductive element outside the tube.

In yet another embodiment a breathing circuit arrangement for removing a moisture of a breathing gas when guiding the breathing gas exhaled and inhaled by a patient includes a tube for the breathing gas exhaled by the patient and a tube for the breathing gas inhaled by the patient. The breathing circuit arrangement for removing a moisture of a breathing gas also includes a branching unit connecting the tube for the breathing gas exhaled by the patient and the tube for the breathing gas inhaled by the patient to airways of the patient, a first one way valve for guiding the breathing gas exhaled through the tube and a second one way valve for guiding the breathing gas inhaled through the tube. The breathing circuit arrangement for removing a moisture of a breathing gas further includes a carbon dioxide remover to remove carbon dioxide from the breathing gas exhaled and an assembly for removing a moisture of the breathing gas by transferring a thermal energy of the breathing gas. The assembly includes a first thermally conductive element in a thermal contact with the breathing gas absorbing a thermal energy of the breathing gas and a medium inside a space being in thermal contact with the first thermally conductive element and which medium is able to absorb and transfer the thermal energy absorbed by the first thermally conductive element. The assembly also includes a second thermally conductive element being in a thermal contact with the medium absorbing the thermal energy transferred by the medium and releasing this thermal energy outside the tubes.

Various other features, objects, and advantages of the invention will be made apparent to those skilled in art from the accompanying drawings and detailed description thereof.

BRIEF DESCRIPTION OF THE DRAWINGS

Figure 1 is a schematic view of a breathing circuit arrangement in an operating environment; and

Figure 2 is a general view of an assembly shown in Figure 1 in accordance with an embodiment.

DETAILED DESCRIPTION OF THE INVENTION

Specific embodiments are explained in the following detailed description making a reference to accompanying drawings. These detailed embodiments can naturally be modified and should not limit the scope of the invention as set forth in the claims.

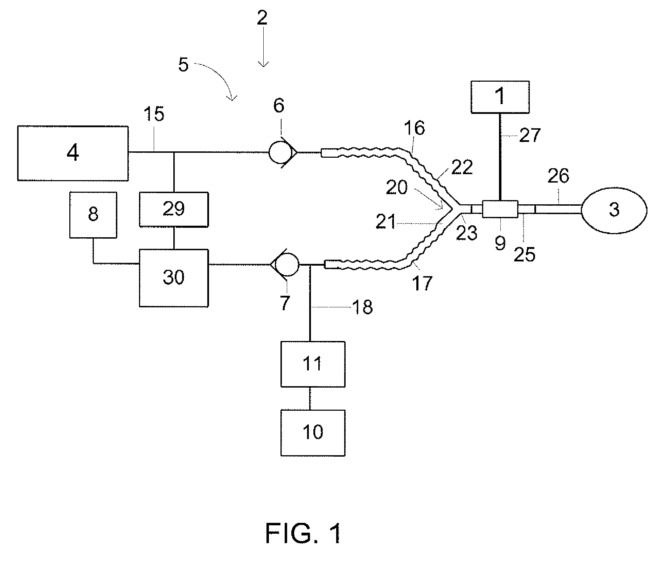

A medical system 2 as shown in Figure 1 may be used in a medical operation when anesthetizing a patient 3. The medical system 2 in this embodiment comprises a medical monitor 1 to control a patient 3, a ventilator 4 to administer a breathing gas to the patient 3 and a breathing circuit arrangement 5, which is between the patient 3 and the ventilator 4 and through which the breathing gas flows. The breathing circuit arrangement 5 of this embodiment is used for guiding the breathing gas exhaled and inhaled by the patient in a re-breathing system. Further the medical system 2 comprises a gas mixer 10 and a vaporizer 11. The gas mixer 10 is for feeding a fresh gas such as oxygen, air and nitrogen oxide into the breathing circuit arrangement 5 along a tube 18. A vaporizer 11 including some anesthetic agent feeds vaporized agent into the breathing circuit arrangement 5 to keep the patient 3 in a desired anesthetic level.

The breathing circuit arrangement 5 of the medical system 2 comprises a first one way valve 6 such as an exhalation valve for guiding the respiration gas through a tube 16 for exhalation gas and a second one way valve 7 such as an inhalation valve for guiding the respiration gas through a tube 17 for inhalation gas and a carbon dioxide remover 8 such as a carbon dioxide absorber to remove or absorb from the exhalation gas carbon dioxide, which carbon dioxide free gas can be returned back to the patient 3. The breathing circuit arrangement 5 also comprises a branching unit 20 such as a Y-piece connecting the tube 16 for the breathing gas exhaled by the patient and the tube 17 for the breathing gas inhaled by the patient to airways of the patient. The branching unit 20 comprises at least three limbs, one of them being an inhalation limb 21 for inspired gas, a second one being an expiration limb 22 for expired gas, a third one being a combined inspiration and expiration limb 23 for both inspired and expired gases. The inhalation limb 21 is connectable to the tube 17 for inhalation and the expiration limb 22 is connectable to the tube 16 for expiration. The combined inspiration and expiration limb 22 of the branching unit 20 may be connectable by means of a patient tube 25 to the endotracheal tube 26 allowing the gas exchange with airways of the subject 4. Also the breathing circuit arrangement 5 may comprise an adapter 9 needed while measuring at least one of a breathing gas flow, pressure, gas concentration of some specific gas component and a composition of the breathing gas. A gas sample may be analyzed by an analyzer (not shown in Figures) connected to the adapter 9 or the gas sample can be withdrawn from the adapter 9 along a tube 27 to the medical monitor 1 including the analyzer.

The breathing circuit arrangement 5 of the medical system comprises also an assembly 30 for removing a moisture in which case typically only a part of the moisture of the breathing gas such as the exhalation gas is removed by transferring a thermal energy of the breathing gas flowing along the tube 16 or the tube 17, which may be components of the breathing circuit arrangement 5, outside these tubes. The assembly 30 may be in various places of the breathing circuit arrangement 5, but preferably between the first one way valve 6 and the second one way valve 7 where the carbon dioxide remover locates, too. Inasmuch as some specific carbon dioxide removers produce moisture it is advantageous to set the assembly 30 downstream the carbon dioxide remover 8, typically between the carbon dioxide remover 8 and the second one way valve 7. But it can be in any place of the medical system where the moisture or at least part of the moisture of the breathing gas can be removed. In Figure 1 a selector valve 29 is arranged in the breathing circuit arrangement 5 upstream the carbon dioxide remover 8, typically between the first one way valve 6 and the assembly 30, to allow the breathing gas either to flow through the carbon dioxide remover or to bypass the carbon dioxide remover. It is, however, advantageous to guide in both cases the breathing gas so that it gets into contact with the assembly 30 for removing the moisture of the breathing gas. Thus for instance the user can manually select whether or not to bypass the carbon dioxide remover or to allow the monitor to do the selection if it is desired.

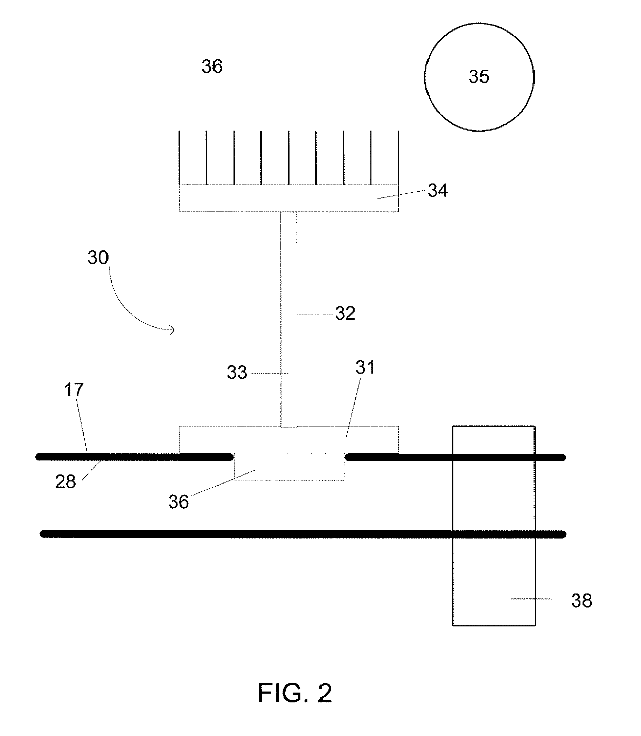

Figure 2 shows details of the assembly 30 for removing the moisture of the breathing gas. A shape of the assembly can naturally vary. The assembly comprises a first thermally conductive element 31, such as a hot interface, in contact with the breathing gas of the tube 17 absorbing a thermal energy of the expiration gas. The first thermally conductive element 31 in contact with the breathing gas of the tube 17 can thus be for example in contact with a surface 28 of the tube or advantageously in direct contact with the breathing gas flowing along the tube 17 to absorb the thermal energy of the breathing gas. The assembly 30 also comprises a medium 33 inside a space 32, which medium is in a thermal contact with the first thermally conductive element 31. The medium 33 is able to absorb of the thermal energy of the first thermally conductive element 31 and transfer by means of a fluid flow to a second thermally conductive element 34, such as a cold interface, which is again in a thermal contact with the medium 33. The second thermally conductive element 34 may be separate from the first conductive element leaving therebetween the medium 33. The thermal contact between the medium and the first thermally conductive element 31 can be direct or indirect. This also applies to the thermal contact between the medium and the second thermally conductive element 32. The second thermally conductive clement 34 is able to absorb the thermal energy transferred by the medium 33. This thermal energy absorbed by the second thermally conductive element 34 is released outside the tube 16, 17 such as an ambient air. The thermal energy transfer from the breathing gas flowing along the tube 16, 17 outside the tube decreases a temperature of the breathing gas.

The assembly 30, such as so called heat pipe, for removing a moisture or at least part of the moisture of the breathing gas is a heat transfer mechanism that combines the principals of both thermal conductivity and matter phase transition to efficiently manage the transfer of heat between two chemical interfaces. The medium 33 is a pressurized fluid when being in contact with the first thermally conductive element 31 and turns into a vapor when absorbing the latent heat of that first thermally conductive element 31, resulting in a phase transition. The vapor naturally flows through the space 33 and condenses back into a liquid when arriving to the second thermally conductive element 34, releasing this latent heat. The liquid then returns to the first thermally conductive element 31 through either capillary action or gravity action where it evaporates again and repeats the cycle continuously until the temperature difference between the breathing gas flowing along the tube 16, 17 is substantially equal with the temperature outside the breathing tube where the second thermally conductive element 34 locates. In addition, the internal pressure of the space 32 can be set or adjusted to facilitate the phase change depending on the demands of the working conditions of the thermally managed system.

The assembly 30 for removing the moisture employs evaporative cooling to transfer the thermal energy from one point to another by the evaporation and condensation of a working fluid or coolant. The assembly 30 for removing the moisture relies on a temperature difference between the ends of the space 32, and cannot lower temperatures at either end beyond the ambient. Thus it should be understood while transferring or absorbing the thermal energy that usually only part of it can be transferred or absorbed. When one end of the space 32 is heated the working medium inside the space at that end evaporates and increases the vapor pressure inside the cavity of the space. The latent heat of evaporation absorbed by the vaporization of the working medium reduces the temperature at the second thermally conductive element.

The vapor pressure over the hot medium at the second thermally conductive element of the assembly 30 is higher than the equilibrium vapor pressure over condensing working medium at the first thermally conductive element 31 of the assembly, and this pressure difference drives a rapid mass transfer to the condensing end where the excess vapor condenses, releases its latent heat, and warms the second thermally conductive element 34 of the assembly.

The conduction is the transfer of the thermal energy such as a heat by a direct contact of particles of a matter. The transfer of the energy could be primarily by elastic impact as in fluids or by free electron diffusion as predominant in metals or a phonon vibration as predominant in insulators. In other words, the heat is transferred by conduction when adjacent atoms vibrate against one another, or as electrons move from one atom to another atom. The conduction is greater in solids, where atoms are in constant contact. In liquids (except liquid metals) and gases, the molecules are usually further apart, giving a lower chance of molecules colliding and passing on the thermal energy.

The law of Heat Conduction, also known as Fourier's law, states that the time rate of the heat transfer through a material is proportional to the negative gradient in the temperature and to the area at right angles, to that gradient, through which the heat is flowing. The differential form of Fourier's Law of the thermal conduction shows that the local heat flux is equal to the product of the thermal conductivity, and the negative local temperature gradient. The heat flux is the amount of energy that flows through a particular surface per unit area per unit time.

Fourier's law in its 1-dimensional form is as follows:

$$q_x{''} = k \frac{\Delta T}{L}$$

The heat flux represents the rate of heat transfer through a section of unit area. The wall heat loss is then

$$q_x = A q_x{''} = -k \nabla T \, ,$$

where $k$ is the thermal conductivity, $\Delta T$ is the temperature difference between the ends, $L$ is the thickness of surface and A is the cross-sectional surface area.

According to one embodiment both absorption and thermal energy releasing areas are optimized. The thermal energy releasing area of the second thermally conductive element 34 should advantageously be larger, more specifically 50 % larger, even more specifically 100% larger than the absorption area of the first thermally conductive element 31.

Plastic is not very good material for conduction. Metals are usually the best conductors of the thermal energy. This is due to the way that metals are chemically bonded: metallic bonds have free-moving electrons, which are able to transfer thermal energy rapidly through the metal. The assembly of the embodiment is a passive device that is constructed in such a way that it acts as though it has extremely high thermal conductivity.

As shown in Figure 2 the assembly may also comprise a fan 35 being close to the second thermally conductive element 34 to increase an efficiency of the transfer of the thermal energy from the second thermally conductive element 34 outside the tube 16. However, cooling works without the fan, too. Further to increase the efficiency of the transfer of the thermal energy the assembly may be equipped with ribs 36 in contact with the first thermally conductive element 31 and the breathing gas flowing along the tubes 16, 17. Also the assembly may comprise the ribs 34 in contact with the second thermally conductive element 36 and outside the breathing gas flowing along the tubes 16, 17. The ribs, which can be parallel, increase an area by which the first thermally conductive element is able to absorb the thermal energy or by which the second thermally conductive element is able to release the thermal energy. The thermal energy releasing area of the second thermally conductive element 34 together with the thermal energy releasing area of those ribs 36 which are in contact with this element 34 should advantageously be larger, more specifically 50 % larger, even more specifically 100% larger than the absorption area of the first thermally conductive element 31 together with the absorption area of those ribs 36 which are in contact with this element 31. However, the ribs are not necessarily needed in the assembly.

By transferring the thermal energy from the breathing gas enables its cooling quickly, whereupon the moisture of the breathing gas is condensing into liquid enabling to collect the condensed liquid in one defined place equipped with a liquid collector 38 which may also be part of the assembly 30. The liquid collector 38 is in fluid connection with the tube 17.

An advantage of the embodiment is that the moisture of the breathing gas can be quickly condensed and collected in one defined place avoiding a need for disconnection of breathing tubes for removing the liquid. This increases a safety for patients and for personnel. The patient can be connected to the breathing circuit arrangement 5 all the time, and ventilation is then unbroken. The personnel do not expose to the anesthesia agent and N20. Also ventilator functionalities work properly, because the resistance issue does not exist. This also increases the patient safety and makes the ventilation more reliable.

The written description uses examples to disclose the invention, including the best mode, and also to enable any person skilled in the art to make and use the invention. The patentable scope of the invention is defined by the claims, and may include other examples that occur to those skilled in the art. Such other examples are intended to be within the scope of the claims if they have structural elements that do not differ from the literal language of the claims, or if they include equivalent structural elements with insubstantial differences from the literal languages of the claims.

## Claims

1. An assembly (30) for removing a moisture of a breathing gas by transferring a thermal energy of said breathing gas, **characterized in that** said assembly comprising:

a first thermally conductive element (31) in a thermal contact with said breathing gas flowing along a tube (16, 17) absorbing a thermal energy of the breathing gas;
a medium (33) inside a space (32) being in thermal contact with said first thermally conductive element and which medium is able to absorb and transfer the thermal energy absorbed by said first thermally conductive element; and
a second thermally conductive element (34) being in a thermal contact with said medium absorbing the thermal energy transferred by said medium and releasing the thermal energy outside said tube.

2. The assembly according to claim 1, **characterized in that** said medium (33) is able to transfer by means of a fluid flow the thermal energy from said first thermally conductive element to said second thermally conductive element.

3. The assembly according to claim 1, **characterized in that** said medium (33) comprises a material being able to a phase transition to transfer the thermal energy.

4. The assembly according to claim 3, **characterized in that** said phase transition is from a liquid to a vapor.

5. The assembly according to claim 3, **characterized in that** said medium (33) is in a liquid form before absorbing a thermal energy of said first thermally conductive element (31) and when absorbing the thermal energy the liquid changes to a vapor form and which vapor flows through said medium inside said space (32) to said second thermally conductive element (34) changing back to the liquid form while releasing the thermal energy to said second thermally conductive element and which liquid is flowing back to said first thermally conductive element.

6. The assembly according to claim 1 further comprising a liquid collector (38) being in fluid connection with said tube for collecting a liquid condensed from the moisture when the thermal energy wase transferred outside said tube.

7. The assembly according to claim 1, **characterized in that** ribs (36) for increasing the efficiency of the transfer of the thermal energy are arranged between said first thermally conductive element (31) and said breathing gas flowing in said tube and/or between said second thermally conductive element (34) and outside said tube.

8. The assembly according to claim 1 further comprising a fan (35) for improving a releasing efficiency of the thermal energy from said second thermally conductive element outside said tube.

9. The assembly according to claim 1, **characterized in that** said second thermally conductive element being in a thermal contact with said medium is also configured to be in contact with outside said tube such as an ambient air.

10. The assembly according to claim 1, **characterized in that** said first thermally conductive element (31) is configured to be in contact with a surface (28) of said tube or advantageously in direct contact with the breathing gas flowing along said tube to absorb the thermal energy of the breathing gas.

11. A method for removing a moisture of a breathing gas by transferring a thermal energy of said breathing gas flowing along a tube (16, 17), **char acterized** in that said method comprising:

absorbing a thermal energy of the breathing gas flowing along said tube into a first thermally conductive element (31);
absorbing from said first thermally conductive element the thermal energy to a medium (33) transferring the thermal energy towards a second thermally conductive element (34);
absorbing from said medium the thermal energy to a second thermally conductive element; and
releasing the thermal energy of said second thermally conductive element outside said tube.

12. The method according to claim 11 further comprising collecting a liquid condensed from the moisture of the breathing gas when the thermal energy of the breathing gas were transferred outside said tube.

13. The method according to claim 11 further comprising ventilating said second thermally conductive element for improving a releasing efficiency of the thermal energy from said second thermally conductive element outside said tube.

14. The method according to claim 11, **characterized in that** when absorbing the thermal energy said medium is going through a phase transition to transfer the thermal energy from said first thermally conductive clement to said second thermally conductive element.

15. A breathing circuit arrangement (5) for removing a moisture of a breathing gas when guiding the breathing gas exhaled and inhaled by a patient, comprising:

a tube (16) for the breathing gas exhaled by the patient;
a tube (17) for the breathing gas inhaled by the patient;

a branching unit (20) connecting said tube (16) for the breathing gas exhaled by the patient and said tube (17) for the breathing gas inhaled by the patient to airways of the patient;

a first one way valve (6) for guiding the breathing gas exhaled through said tube (16);

a second one way valve (7) for guiding the breathing gas inhaled through said tube (17);

a carbon dioxide remover (8) to remove carbon dioxide from the breathing gas exhaled; and

an assembly (30) for removing a moisture of the breathing gas by transferring a thermal energy of said breathing gas, **chara cterized** in that said assembly comprising:

a first thermally conductive element (31) in a thermal contact with said breathing gas absorbing a thermal energy of the breathing gas;

a medium (33) inside a space (32) being in thermal contact with said first thermally conductive element and which medium is able to absorb and transfer the thermal energy absorbed by said first thermally conductive element; and

a second thermally conductive element (34) being in a thermal contact with said medium absorbing the thermal energy transferred by the medium and releasing this thermal energy outside said tubes.

FIG. 1

FIG. 2

Europäisches
Patentamt

European
Patent Office

Office européen
des brevets

# EUROPEAN SEARCH REPORT

**Application Number**

EP 09 17 9796

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 01/49351 A2 (INSTRUMENTARIUM CORP [FI])<br>12 July 2001 (2001-07-12)<br>* page 4, line 5 - page 6, line 27 *<br>* figures 1-2 *<br>----- | 1,6-13,<br>15 | INV.<br>A61M16/10 |
| X | US 2006/037613 A1 (KWOK PHILIP R [AU] ET<br>AL KWOK PHILIP RODNEY [AU] ET AL)<br>23 February 2006 (2006-02-23)<br>* paragraph [0043] *<br>* paragraph [0064] *<br>* paragraphs [0083] - [0086] *<br>* figures 1, 3 *<br>----- | 1-6,<br>9-12,14 | |
| X | US 3 592 191 A (JACKSON RICHARD R)<br>13 July 1971 (1971-07-13)<br>* column 2, line 64 - column 4, line 39 *<br>* figures 1,2 *<br>----- | 1,6,<br>9-12,15 | |
| X | DE 20 2008 008519 U1 (LEVINA DINA [DE])<br>23 July 2009 (2009-07-23)<br>* paragraphs [0048] - [0049]; figure 2 *<br>----- | 1-3,6,<br>9-12,14 | TECHNICAL FIELDS SEARCHED (IPC)<br><br>A61M |

The present search report has been drawn up for all claims

| Place of search | Date of completion of the search | Examiner |
|---|---|---|
| The Hague | 12 May 2010 | Schlaug, Martin |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 09 17 9796

This annex lists the patent family members relating to the patent documents cited in the above-mentioned European search report.
The members are as contained in the European Patent Office EDP file on
The European Patent Office is in no way liable for these particulars which are merely given for the purpose of information.

12-05-2010

| Patent document cited in search report | | Publication date | Patent family member(s) | | Publication date |
|---|---|---|---|---|---|
| WO 0149351 | A2 | 12-07-2001 | AU 1879601 A | | 16-07-2001 |
| | | | EP 1181070 A2 | | 27-02-2002 |
| | | | US 6523538 B1 | | 25-02-2003 |
| US 2006037613 | A1 | 23-02-2006 | US 2009174092 A1 | | 09-07-2009 |
| US 3592191 | A | 13-07-1971 | NONE | | |
| DE 202008008519 | U1 | 23-07-2009 | NONE | | |

EPO FORM P0459

For more details about this annex : see Official Journal of the European Patent Office, No. 12/82